# EUROPEAN PATENT APPLICATION

(11) **EP 3 222 241 A1**
(43) Date of publication of application: **27.09.2017**
(21) Application number: 15861822.3
(22) Date of filing: 02.11.2015
(51) Int. Cl.: A61B 18/14

(54) **ENDOSCOPIC TREATMENT INSTRUMENT**

(30) Priority: 18.11.2014 JP 2014233508
(71) Applicant: Olympus Corporation, Hachioji-shi, Tokyo 192-8507 (JP)
(72) Inventor: ITO, Takashi, Hachioji-shi, Tokyo 192-8507 (JP); MOTOSUGI, Shunsuke, Hachioji-shi, Tokyo 192-8507 (JP); WAKE, Fuminori, Hachioji-shi, Tokyo 192-8507 (JP); YAMAMOTO, Tetsuya, Hachioji-shi, Tokyo 192-8507 (JP)
(74) Representative: Gunzelmann, Rainer
(86) International application number: PCT/JP2015/080920
(87) International publication number: WO 2016/080180

(57) **Abstract**

An endoscopic treatment tool includes: a tubular insertion part; a stopper having a through hole and provided at a distal end part of the insertion part; a manipulation wire inserted through the insertion part to be advanceable and retractable; a knife part provided at a distal end part of the manipulation wire and inserted through the through hole; and a restriction member provided closer to a proximal end side than the stopper and connecting the knife part and the manipulation wire. The stopper has an inner surface which is in surface contact with a part of the restriction member. When the restriction member is pressed against the stopper, the knife part is locked not to be rotated with respect to the insertion part, the restriction member and the stopper have a non-contact region at at least a part thereof in a direction about the axis of the insertion part, and the restriction member is in surface contact with the stopper such that the non-contact region is located entirely in an axial direction of the insertion part in a region at which the inner surface is disposed.

## Description

### Technical Field

The present invention relates to an endoscopic treatment tool, and more particularly, to an endoscopic treatment tool with which a rotational manipulation of a site to perform treatment and liquid feeding can be performed.

Priority is claimed on Japanese Patent Application No. 2014-233508, filed November 18, 2014, the content of which is incorporated herein by reference.

### Background Art

In the related art, an endoscopic treatment tool including a knife part endoscopically inserted into a body cavity and configured to resect mucosa or the like by applying a high frequency current is well known (for example, refer to Patent Literature 1). Such an endoscopic treatment tool is constituted with the knife part used for treatment attached to a distal end of a wire inserted through an insulating sheath inserted into a channel of an endoscope. The knife part can protrude or retract from a distal end of the sheath through manipulation of a manipulation member to which a proximal end of the wire is fixed.

As the shape of an incision part, a round-rod-shaped member, a so-called hook knife in which a distal end of a rod-shaped member is bent in an L shape, or the like is known.

In an endoscopic treatment tool disclosed in Patent Literature 1, when a stopper protrusion provided at a high frequency knife is pressed against a rigid cylindrical body attached to a distal end part of a flexible sheath, a tapered surface of the rigid cylindrical body comes in surface contact with the stopper protrusion and thus the high frequency knife is stably held on a central axis of the flexible sheath.

### Citation List

### Patent Literature

Patent Literature 1: Japanese Unexamined Patent Application, First Publication No. 2007-44393

### Summary of Invention

### Technical Problem

In the case of a hook knife, a knife part is rotated with respect to a sheath so that a direction of a distal end part of the knife part is changed. Thus, a user rotates a wire, adjusts the direction of the distal end part of the knife part in a state appropriate for a procedure, and uses the hook knife. The direction of the distal end part of the knife part can preferably be held (locked) after the direction of the distal end part of the knife part is adjusted.

Also in the case of a structure like the endoscopic treatment tool disclosed in Patent Literature 1, the stopper protrusion is strongly pressed against the tapered surface so that the high frequency knife and the rigid cylindrical body can be held such that the high frequency knife and the rigid cylindrical body are not relatively rotated.

Patent Literature 1 discloses that, also in a fully protruding state in which the stopper protrusion is in contact with the proximal end part of the rigid cylindrical body, a communication path for liquid feeding or suctioning is formed at an inner diameter part of the rigid cylindrical body. However, in order to hold the knife part such that the knife part is not rotated relative to the rigid cylindrical body, a mechanism at the knife part side such as the stopper protrusion needs to be very strongly pressed against a mechanism at the sheath side such as the rigid cylindrical body. Since a dimension of the knife part of the endoscopic treatment tool in a radial direction thereof is generally very small at about several millimeters, even in the case of the structure like the endoscopic treatment tool disclosed in Patent Literature 1, there is a possibility that liquid feeding or the like is actually not sufficiently performed in a state in which the high frequency knife is strongly pressed against the rigid cylindrical body such that the high frequency knife and the rigid cylindrical body are not relatively rotated.

The present invention was made in view of the above-described circumstances, and the present invention is for the purpose of providing an endoscopic treatment tool in which locking of a site used for treatment and liquid feeding or the like can be simultaneously and reliably performed.

### Solution to Problem

According to a first aspect of the present invention, an endoscopic treatment tool includes: a tubular insertion part; a stopper having a through hole and provided at a distal end part of the insertion part; a manipulation wire inserted through the insertion part to be advanceable and retractable with respect to the insertion part; a treatment part provided at a distal end part of the manipulation wire and inserted through the through hole; and a restriction member provided closer to a proximal end side than the stopper inside the insertion part, the restriction member connecting the treatment part and the manipulation wire. The stopper has a locking surface which is in surface contact with a part of the restriction member. When the restriction member is pressed against the stopper, the treatment part is locked to the insertion part such that the treatment part is not rotated about an axis of the insertion part, the restriction member and the stopper have a non-contact region at at least a part thereof in a direction about the axis of the insertion part, and the restriction member is in surface contact with the stopper such that the non-contact region is located in an axial direction of the insertion part in a region at which the locking surface is disposed.

According to a second aspect of the present invention, the endoscopic treatment tool according to the first aspect may further include a flow path surface provided at at least one of the restriction member and the stopper. The non-contact region may be formed at a site at which the flow path surface is disposed when the restriction member is pressed against the stopper.

According to a third aspect of the present invention, an endoscopic treatment tool includes: a tubular insertion part; a stopper having a through hole and provided at a distal end part of the insertion part; a manipulation wire inserted through the insertion part to be advanceable and retractable with respect to the insertion part; a treatment part provided at a distal end part of the manipulation wire and inserted through the through hole; and a restriction member provided closer to a proximal end side than the stopper inside the insertion part, the restriction member connecting the treatment part and the manipulation wire. The stopper has a locking surface which is in surface contact with a part of the restriction member. The restriction member has a flow path formed such that a first opening of the flow path is open at a distal end part of the restriction member and a second opening of the flow path is open at a position closer to a proximal end side than a site which is in surface contact with the locking surface. The treatment part is locked to the insertion part such that the treatment is not rotated about an axis of the manipulation wire when the restriction member is pressed against the stopper.

According to a fourth aspect of the present invention, in the endoscopic treatment tool according to the first aspect, the insertion part may have a resinous tube sheath and a metallic coil sheath disposed inside the tube sheath.

### Advantageous Effects of Invention

According to the above aspects, locking of the site used for treatment and liquid feeding or the like can be simultaneously and reliably performed.

### Brief Description of Drawings

Fig. 1 is a view showing an overall constitution of an endoscopic treatment tool according to a first embodiment of the present invention.
Fig. 2 is an exterior diagram of a restriction member of the endoscopic treatment tool.
Fig. 3 is an exterior diagram of the restriction member.
Fig. 4 is a partially enlarged cross-sectional view of a manipulation part of the endoscopic treatment tool.
Fig. 5 is an enlarged cross-sectional view showing a joining part between a manipulation wire and a pipe.
Fig. 6 is a view showing one form when the endoscopic treatment tool is used.
Fig. 7 is a view showing another example of the restriction member.
Fig. 8 is a cross-sectional view showing a distal end part of an endoscopic treatment tool according to a second embodiment of the present invention.
Fig. 9 is a cross-sectional view showing a distal end part of an endoscopic treatment tool in a modified example of the endoscopic treatment tool.
Fig. 10 is a cross-sectional view showing a manipulation part of an endoscopic treatment tool according to a third embodiment of the present invention.
Fig. 11 is a cross-sectional view showing a constitution example of a distal end part of the endoscopic treatment tool.
Fig. 12 is a cross-sectional view showing a distal end part of an endoscopic treatment tool in a modified example of the endoscopic treatment tool of the present invention.
Fig. 13 is a view showing another example of a knife part.
Fig. 14 is a view showing another constitution example of a stopper.
Fig. 15 is a view showing yet another constitution example of the stopper.

### Description of Embodiments

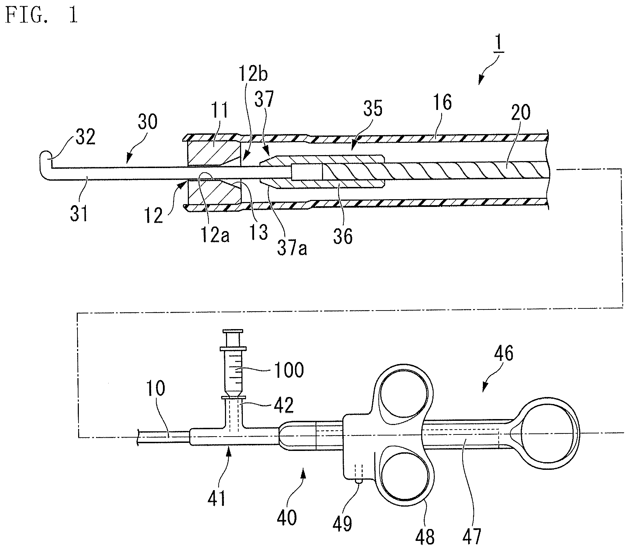

A first embodiment of the present invention will be described with reference to Figs. 1 to 6.

Fig. 1 is a view showing an overall constitution of an endoscopic treatment tool 1 of this embodiment. The endoscopic treatment tool 1 is inserted into an endoscope including a flexible insertion part and used. The endoscopic treatment tool 1 includes a tubular insertion part 10 with flexibility, a manipulation wire 20 inserted through the insertion part 10, a knife part (a treatment part) 30 attached to a distal end part of the manipulation wire 20, and a manipulation part 40 connected to the insertion part 10.

The insertion part 10 is constituted of an elongated sheath 16 with flexibility. A well-known resinous tube sheath or the like can be used as the sheath 16. The manipulation wire 20 is disposed inside the sheath 16. The knife part 30 is provided at the distal end part of the manipulation wire 20. A proximal end part of the manipulation wire 20 is connected to the manipulation part 40, and the manipulation wire 20 and the knife part 30 can be advanced and retracted with respect to the insertion part 10 by manipulating the manipulation part 40. A well-known stranded wire or the like can be used as the manipulation wire 20.

The knife part 30 is a so-called hook knife formed of a metal or the like, and has a main body part 31 connected to the manipulation wire 20 and a bending part 32 provided at a distal end side of the main body part 31. The bending part 32 is bent about 90° with respect to the main body part 31 and extends in a direction away from an axis of the insertion part 10. An angle formed by the bending part 32 and the main body part 31 is not particularly limited to 90° and may be appropriately set.

The manipulation part 40 has a fluid supply part 41 connected to the insertion part 10 and a wire manipulation part 46 for manipulating the manipulation wire. The fluid supply part 41 has a cock 42. A syringe 100 or the like is connected to the cock 42 so that a fluid such as a liquid or a gas can be supplied from the fluid supply part 41 into the insertion part 10. A suction source is connected to the cock 42 so that a liquid or the like in a patient's body can also be suctioned through a distal end of the insertion part 10.

The wire manipulation part 46 has a well-known constitution which includes a rod-shaped manipulation main body 47 and a slider 48 slidably attached to the manipulation main body 47. The proximal end part of the manipulation wire 20 is attached to the slider 48 through a pipe which will be described later. The slider 48 slides with respect to the manipulation main body 47 so that the manipulation wire 20 can be advanced and retracted with respect to the insertion part 10. A plug 49 to be connected to an external power supply is provided at the slider 48.

A power supply cord or the like is connected to the plug 49 so that electric power can be fed to the knife part 30 from the plug via the manipulation wire 20.

As shown in Fig. 1, a stopper 11 for restricting a protruding length of the knife part 30 from the insertion part 10 to a predetermined value or less is attached to the distal end part of the insertion part 10. The stopper 11 is a substantially cylindrical member, and a through hole 12 is provided in the stopper 11 along a central axis of the cylindrical shape. The through hole 12 has a first region 12a at a distal end side thereof and a second region 12b at a proximal end side thereof, and the knife part 30 is inserted through the through hole 12. An inner diameter of the first region 12a is constant in a central axial direction of the stopper 11. An inner diameter of the second region 12b gradually increases from a distal end side of the stopper 11 toward a proximal end side of the stopper 11. An inner surface 13 of the second region 12b is a locking surface which comes in surface contact with a restriction member 35 when relative movement of the knife part 30 is suppressed. This will be described later in detail, but hereinafter, the inner surface 13 is referred to as "a locking surface 13" in some cases.

The knife part 30 and the manipulation wire 20 are integrally connected to each other by the restriction member 35 at an internal region of the insertion part 10 which is closer to the proximal end side than the stopper 11. The restriction member 35 is a cylindrical member, and has a base part 36 of which a diameter dimension is constant in an axial direction thereof and a tapered reduced diameter part 37 which is provided closer to the distal end side than the base part 36 and of which a diameter dimension gradually decreases. The locking surface 13 of the stopper 11 and an outer peripheral surface 37a of the reduced diameter part 37 are parallel (including substantially parallel) with each other in a state in which the stopper 11 and the restriction member 35 are disposed to be coaxial with each other.

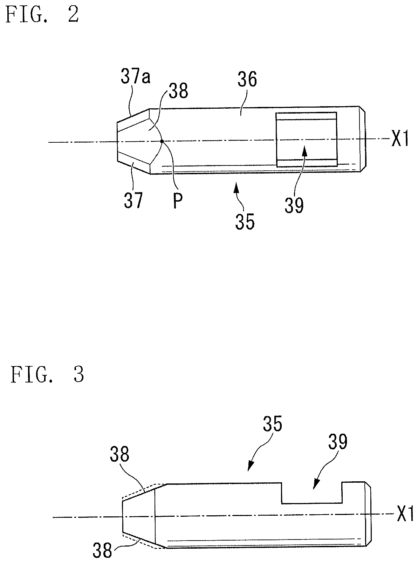

Fig. 2 is a view showing an outer peripheral surface of the restriction member 35 with an orientation shown in Fig. 1. As shown in Fig. 2, a part of the base part 36 and the reduced diameter part 37 is cut out so that a flow path surface 38 is formed at the restriction member 35. The flow path surface 38 is a plane, and includes a distal end of the restriction member 35 and a point P on an outer peripheral surface of the base part 36 located closer to the proximal end side than the reduced diameter part 37. Thus, if positions of the restriction member 35 in a direction of an axis X1 are the same, the distance between the flow path surface 38 and the axis X1 is shorter than the distance between the outer peripheral surface 37a and the axis X1. As shown in Fig. 3, two flow path surfaces 38 are formed opposite each other to surround the axis X1.

Fig. 3 is a view of the restriction member 35 when the restriction member 35 is viewed from a lower side in Fig. 1. Reference numeral 39 in Figs. 2 and 3 is a hole into which a brazing material or the like for connecting the manipulation wire 20 and the knife part 30 is poured.

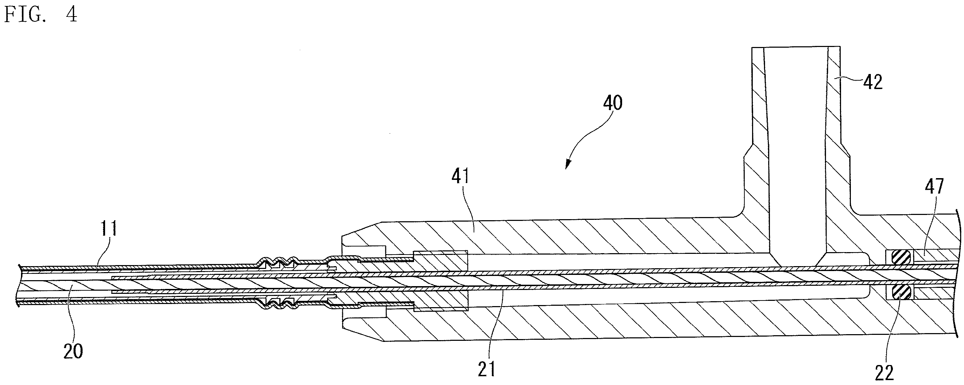

Fig. 4 is a partial cross-sectional view of the manipulation part 40. A proximal end side of the manipulation wire 20 is inserted through a pipe 21 for preventing buckling and is fixed to the pipe 21. The pipe 21 extends to an inside of the manipulation main body 47 through the fluid supply part 41 and is fixed to the slider 48 (not shown). Water tightness of a space between the fluid supply part 41 and the pipe 21 is secured by an O ring 22, and a fluid supplied from the cock 42 does not leak to the wire manipulation part 46.

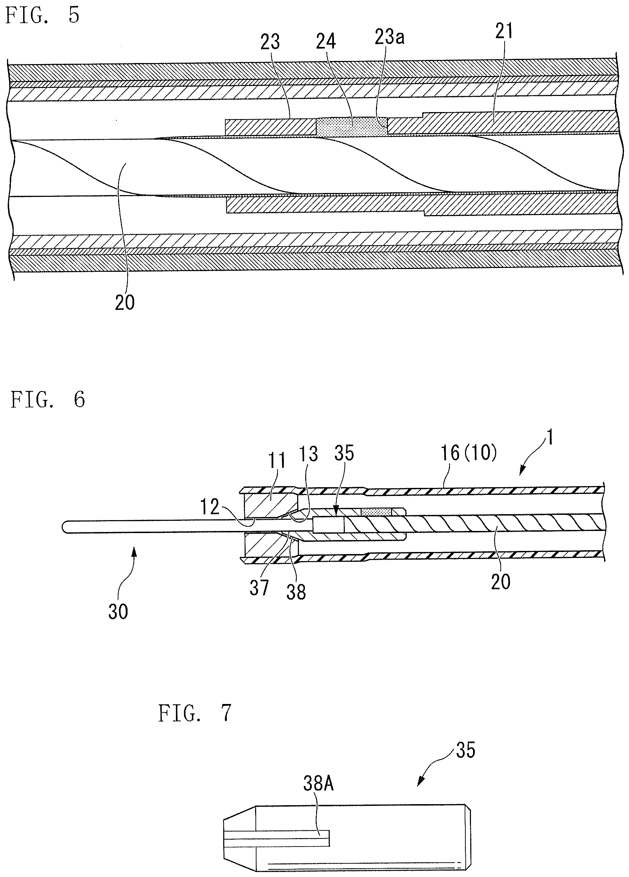

As shown in the enlarged view of Fig. 5, a small diameter part 23 of which a diameter dimension is formed to be smaller than a region of a proximal end side of the pipe 21 by means of cutting or the like is provided at a distal end part of the pipe 21. The manipulation wire 20 and the pipe 21 are integrally connected to each other by a brazing material 24 which has been poured through a hole 23a provided at the small diameter part 23. Such a structure prevents water or the like from entering the inside of the pipe 21 from a distal end side of the pipe 21. Furthermore, the structure hardly causes the brazing material 24 bulging from the hole 23 a to protrude outward in a radial direction thereof from a region of the pipe 21 which is closer to the proximal end side than the small diameter part 23, and thus a situation in which a step of removing a surplus brazing material after brazing is needed hardly occurs in the structure.

A motion when the endoscopic treatment tool 1 of this embodiment constituted as described above is used will be described.

First, a user inserts an endoscopic insertion part into the patient's body, and moves a distal end part of the endoscopic insertion part to the vicinity of a site to be treated. Subsequently, the user inserts the endoscopic treatment tool 1 from the distal end side at which the knife part 30 is provided into a forceps plug or the like of the endoscope, and inserts the endoscopic treatment tool 1 through a treatment tool channel in the endoscopic insertion part. Insertion of the endoscopic treatment tool 1 through the channel may be performed before the endoscopic insertion part is inserted into the patient's body.

When the user advances a slider 48 with respect to the manipulation main body 47, the knife part 30 protrudes from the distal end of the insertion part 10. As described above, the insertion part 10 is fixed to the fluid supply part 41 and the manipulation wire 20 is fixed to the wire manipulation part 46. Therefore, the entire wire manipulation part 46 is rotated with respect to the fluid supply part 41 so that the manipulation wire 20 and the knife part 30 are rotated with respect to the insertion part 10 and thus a direction of the bending part 32 can be adjusted to a desired direction.

When the user wants to fix (lock) the direction of the bending part 32 in a state in which the direction of the bending part 32 is adjusted, the user strongly advances the slider 48. Thus, the restriction member 35 is pressed against the stopper 11. Since the locking surface 13 and the outer peripheral surface 37a are parallel to each other as described above, substantially the entire outer peripheral surface 37a is in surface contact with the locking surface 13 when the slider 48 is manipulated to be advanced. As a result, the stopper 11 is engaged with the restriction member 35 due to a frictional force occurring between the outer peripheral surface 37a and the locking surface 13, and thus the knife part 30 is locked to the insertion part 10 not to rotate about the axis of the insertion part 10.

Fig. 6 is a cross-sectional view showing a state of a distal end part of the endoscopic treatment tool 1 when the distal end part thereof is viewed from the lower side in Fig. 1. The two flow path surfaces 38 provided at the restriction member 35 are at positions closer to the axis X1 than the outer peripheral surface 37a in a state in which the stopper 11 is in surface contact with and frictionally engaged with the restriction member 35 as described above. Thus, the two flow path surfaces 38 are not in contact with the locking surface 13. As a result, the stopper 11 is not in contact with the restriction member 35 at the reduced diameter part 37 over the entire circumference of the restriction member 35, and the stopper 11 is locked to the restriction member 35 in a state in which a non-contact region is secured at a part of the reduced diameter part 37 provided with the flow path surface 38 in a direction about the axis of the insertion part. As shown in Fig. 6, the non-contact region is continuous in a region provided with the locking surface 13 in an axial direction of the insertion part 10. Therefore, a space defined by a part of the locking surface 13 and the flow path surface 38 is secured to be continuous in the axial direction of the insertion part 10 in a state in which the stopper 11 is frictionally engaged with the restriction member 35. Thus, a fluid introduced from the cock 42 enters the space and can preferably flow through a gap between an inner circumferential surface of the through hole 12 and the knife part 30 inside the through hole 12 of the stopper 11.

With the above-described action, the endoscopic treatment tool 1 can perform liquid feeding or the like from the distal end part of the insertion part 10 while the knife part 30 is locked to the insertion part 10. Note that a liquid fed from the distal end part of the insertion part 10 includes water, physiological saline, a fluid with viscosity, and the like.

As described above, according to the endoscopic treatment tool 1 of this embodiment, since the flow path surface 38 is formed by cutting a part of the outer peripheral surface 37a of the restriction member 35 which is in surface contact with the stopper 11, a flow path for supplying the fluid can be reliably opened while the surface contact between the stopper 11 and the restriction member 35 is secured so that a locking function of the knife part 30 is reliably exhibited. Therefore, the locking function of the knife part and the liquid feeding or the like can be appropriately and reliably compatible.

In this embodiment, the number of flow path surfaces formed at the restriction member is not particularly limited and any number that is at least one or more may be adopted. Furthermore, the shape of the flow path surface is not limited to the above-described plane. The shape of the flow path surface may be a groove 38A of which a cross section has a V shape as shown in Fig. 7 or may be a curved surface.

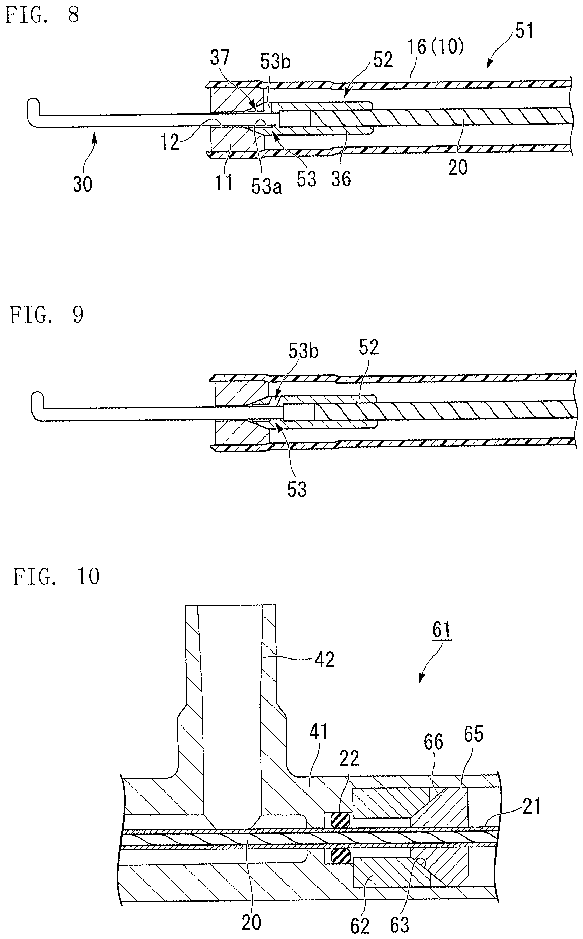

A second embodiment of the present invention will be described with reference to Figs. 8 and 9. An endoscopic treatment tool 51 of this embodiment and the above-described endoscopic treatment tool 1 differ from each other in that a flow path for supplying a fluid is formed in a restriction member. In the following description, constituent elements which are the same as those which have already been described are denoted with the same reference numerals and descriptions thereof will be omitted.

Fig. 8 is a cross-sectional view showing a distal end part of the endoscopic treatment tool 51. An outer shape of a restriction member 52 is substantially the same as that of the restriction member 35 of the first embodiment. A flow path 53 into which the fluid can be introduced is formed inside the restriction member 52.

The flow path 53 has a first region 53a formed to be substantially coaxial with a central axis of the restriction member 52 and a second region 53b formed to be substantially perpendicular to the central axis of the restriction member 52. The first region 53a is open at a distal end of the restriction member 52, and the knife part 30 is inserted through the first region 53a. The second region 53b is open at an outer peripheral surface of the base part 36 at a position closer to the proximal end side than the reduced diameter part 37. Since the first region 53a communicates with the second region 53b, the fluid supplied inside the insertion part 10 enters the flow path 53 from the second region 53b and flows out from the distal end of the restriction member 52 through the first region 53a when a pressure is applied to the fluid. After that, the fluid flows out from the distal end of the insertion part 10 through the through hole 12 of the stopper 11.

The second region 53b is open at the position of the restriction member 52 which is closer to the proximal end side than the reduced diameter part 37. Thus, even if the user brings the stopper 11 into surface contact with the restriction member 52 in order to lock the knife part 30, an opening of the second region 53b is not covered with a locking surface. Therefore, the user can preferably perform liquid feeding or the like from the flow path 53 while the knife part 30 is locked.

Also, in this embodiment, a part of an outer peripheral surface of the reduced diameter part which is frictionally engaged with the stopper may not be cut. Thus, a locking function can be stabilized.

In addition, the entire outer peripheral surface of the reduced diameter part is in surface contact with the locking surface. Thus, the stopper 11 and the restriction member 52 are reliably coaxial with each other when the stopper 11 is locked to the restriction member 52, and thus axis misalignment of the knife part 30 can be suppressed.

In this embodiment, the second region may not be provided to be perpendicular to the central axis of the restriction member. For example, as in a modified example shown in Fig. 9, the opening of the second region 53b may be set at a position closer to a proximal end of the restriction member 52, and the central axis of the restriction member 52 and the second region 53b may be formed to have an obtuse angle at a distal end side of the restriction member 35. Thus, the fluid can more easily flow through the flow path 53.

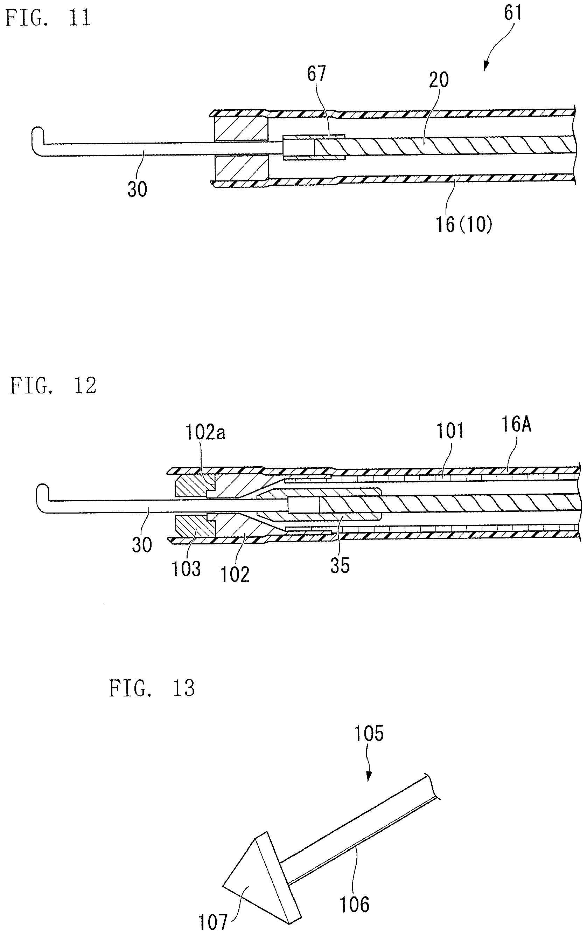

A third embodiment of the present invention will be described with reference to Figs. 10 and 11. An endoscopic treatment tool of this embodiment and the endoscopic treatment tools of the above-described embodiments differ from each other in that a mechanism for locking a knife part is provided at a manipulation part.

Fig. 10 is an enlarged cross-sectional view showing a fluid supply part 41 of an endoscopic treatment tool 61 of this embodiment. A stopper 62 having a locking surface 63 is disposed at a proximal end part of the fluid supply part 41.

A restriction member 65 having a reduced diameter part 66 is fixed to the pipe 21. Therefore, when the slider 48 (not shown) is advanced, the pipe 21 and the restriction member 65 advance with respect to the fluid supply part 41 and are pressed against the locking surface 63 of the stopper 62. Thus, the knife part 30 can be locked to the insertion part 10.

Fig. 11 shows an example of a distal end constitution of the endoscopic treatment tool 61. The endoscopic treatment tool 61 need not include a mechanism for locking the knife part 30 in a distal end part of the insertion part 10. Thus, the knife part 30 may be connected to the manipulation wire 20 through caulking, brazing, or the like using a normal pipe 67 or the like.

Although the endoscopic treatment tools of the present invention have been described above using the embodiments, the technical scope of the present invention is not limited to the above-described embodiments. In addition, a combination of constituent elements can be changed, various changes can be added to various constituent elements, or various constituent elements can be omitted without departing from the gist of the present invention.

For example, as in a modified example shown in Fig. 12, when the insertion part 10 is formed to include a resinous tube sheath 16A and a metallic coil sheath 101 and a stopper 102 is formed of a conductor, current leakage may be prevented by disposing an insulating member 103 such as a ceramic at a distal end side of the stopper 102. In such a constitution, if a groove 102a is provided at the stopper 102 and the insulating member 103 is disposed such that a part of the insulating member 103 is engaged with the groove 102a, it is preferable because the insulating member 103 is not shifted in a radial direction of the insertion part 10 even if an external force is applied to the distal end part of the insertion part 10.

In a constitution of this modified example, rigidity of the insertion part 10 is increased due to the coil sheath 101 disposed inside the tube sheath 16A. In addition, in this modified example, a proximal end part of the stopper 102 is fixed to the coil sheath 101. With these constitutions, even if the restriction member 35 is strongly pressed against the stopper 102 in order to lock the knife part 30, the coil sheath 101 and the stopper 102 reliably receive a pressing force occurring due to a pressing manipulation. As a result, a situation in which a locking function is not sufficiently exhibited due to a case in which the tube sheath 16A extends by the pressing force so that the stopper 102 advances can be appropriately prevented. This effect can be exhibited regardless of the above-described disposition aspects of the insulating member 103.

Also, the shape of the treatment part is not limited to the above-described shape, and any shape that needs to be rotated may be adopted. For example, like a knife part 105 shown in Fig. 13, a triangular plate-like chip 107 may be attached to a distal end of a main body part 106 in a front view. The shape of the chip 107 in the front view is not limited to a triangular shape and may be other shapes such as a star shape.

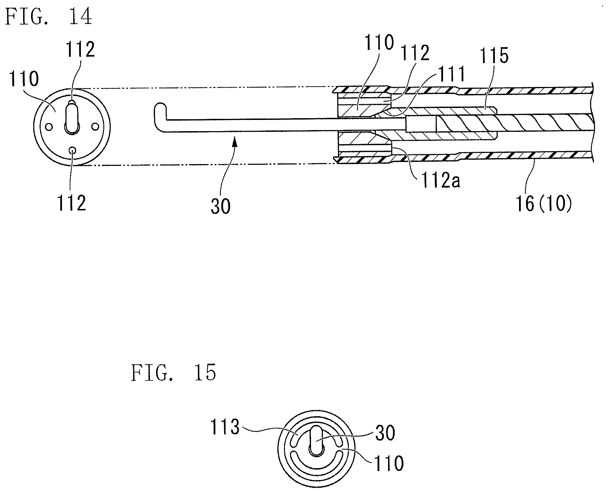

Although slightly different from the above-described constitution, as shown in Fig. 14, a locking function and liquid feeding or the like can be compatible also by providing a flow path 112 at a stopper 110 separately from a locking surface 111. In this case, members constituting a distal end part of the insertion part 10 may be designed such that an opening 112a of the flow path 112 at the proximal end side is not formed at the locking surface 111 and the opening 112a is not blocked by a restriction member 115 when the restriction member 115 is pressed against the stopper 110.

In the above-described aspects, the shape of the flow path formed at the stopper 110 is not particularly limited. In addition, for example, like a flow path 113 shown in Fig. 15, the shape thereof may be a shape extending in a circumferential direction of the stopper 110 in the front view.

In addition, the flow path surface may be provided at the stopper. For example, even in a case where a groove is formed at the above-described locking surface in the axial direction of the insertion part and an inner surface of the groove is set as the flow path surface, a flow path can be secured between the groove and an outer peripheral surface of the restriction member in a locked state in which the restriction member is in surface contact with the stopper.

### Industrial Applicability

According to the above-described embodiments, locking of a site used for treatment and liquid feeding or the like can be simultaneously and reliably performed.

### Reference Signs List

- 1, 51:: endoscopic treatment tool
- 10:: insertion part
- 11, 102:: stopper
- 12:: through hole
- 13:: inner surface (locking surface)
- 16A:: tube sheath
- 20:: manipulation wire
- 30:: knife part (treatment part)
- 35, 52:: restriction member
- 38:: flow path surface
- 38A:: groove (flow path surface)
- 53:: flow path
- 101:: coil sheath

## Claims

1. An endoscopic treatment tool comprising:
a tubular insertion part;
a stopper having a through hole and provided at a distal end part of the insertion part;
a manipulation wire inserted through the insertion part to be advanceable and retractable with respect to the insertion part;
a treatment part provided at a distal end part of the manipulation wire and inserted through the through hole; and
a restriction member provided closer to a proximal end side than the stopper inside the insertion part, the restriction member connecting the treatment part and the manipulation wire,
wherein the stopper has a locking surface which is in surface contact with a part of the restriction member, and
wherein, when the restriction member is pressed against the stopper, the treatment part is locked to the insertion part such that the treatment part is not rotated about an axis of the insertion part, the restriction member and the stopper have a non-contact region at at least a part thereof in a direction about the axis of the insertion part, and the restriction member is in surface contact with the stopper such that the non-contact region is located entirely in an axial direction of the insertion part in a region at which the locking surface is disposed.

2. The endoscopic treatment tool according to claim 1, further comprising:
a flow path surface provided at at least one of the restriction member and the stopper,
wherein the non-contact region is formed at a site at which the flow path surface is disposed when the restriction member is pressed against the stopper.

3. An endoscopic treatment tool comprising:
a tubular insertion part;
a stopper having a through hole and provided at a distal end part of the insertion part;
a manipulation wire inserted through the insertion part to be advanceable and retractable with respect to the insertion part;
a treatment part provided at a distal end part of the manipulation wire and inserted through the through hole; and
a restriction member provided closer to a proximal end side than the stopper inside the insertion part, the restriction member connecting the treatment part and the manipulation wire,
wherein the stopper has a locking surface which is in surface contact with a part of the restriction member,
the restriction member has a flow path formed such that a first opening of the flow path is open at a distal end part of the restriction member and a second opening of the flow path is open at a position closer to a proximal end side than a site which is in surface contact with the locking surface, and
the treatment part is locked to the insertion part such that the treatment is not rotated about an axis of the manipulation wire when the restriction member is pressed against the stopper.

4. The endoscopic treatment tool according to claim 1, wherein the insertion part has a resinous tube sheath and a metallic coil sheath disposed inside the tube sheath.
